(19) Europäisches Patentamt / European Patent Office / Office européen des brevets

(11) **EP 3 463 206 B1**

(12) **EUROPÄISCHE PATENTSCHRIFT**

(45) Veröffentlichungstag und Bekanntmachung des Hinweises auf die Patenterteilung:
**09.10.2024 Patentblatt 2024/41**

(21) Anmeldenummer: **17733977.7**

(22) Anmeldetag: **01.06.2017**

(51) Internationale Patentklassifikation (IPC):
***A61F 2/90*** *(2013.01)*

(52) Gemeinsame Patentklassifikation (CPC):
**A61F 2/90; A61F 2/95;** A61F 2210/0014;
A61F 2230/0006; A61F 2230/0019;
A61F 2230/0091; A61F 2250/0015;
A61F 2250/0036; A61F 2250/0039;
A61F 2250/0067; A61F 2250/0098

(86) Internationale Anmeldenummer:
**PCT/EP2017/063302**

(87) Internationale Veröffentlichungsnummer:
**WO 2017/207689 (07.12.2017 Gazette 2017/49)**

(54) **VASOSPASMUSBEHANDLUNG**

VASOSPASM TREATMENT

TRAITEMENT D'UN ANGIOSPASME

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.06.2016 DE 102016110199**

(43) Veröffentlichungstag der Anmeldung:
**10.04.2019 Patentblatt 2019/15**

(73) Patentinhaber:
• **Charité - Universitätsmedizin Berlin**
**10117 Berlin (DE)**
• **femtos GmbH**
**44799 Bochum (DE)**

(72) Erfinder:
• **LIEBIG, Thomas**
**81827 München (DE)**
• **MONSTADT, Hermann**
**44797 Bochum (DE)**
• **HENKES, Hans**
**70192 Stuttgart (DE)**
• **HANNES, Ralf**
**44137 Dortmund (DE)**

(74) Vertreter: **Schneiders & Behrendt Bochum**
**Gerard-Mortier-Platz 6**
**44793 Bochum (DE)**

(56) Entgegenhaltungen:
EP-A1- 1 604 697     DE-A1- 10 301 850
US-A1- 2016 081 825

**Beschreibung**

Die Erfindung betrifft eine Vorrichtung mit einer Stentstruktur, die zur Einbringung in Blutgefäße des menschlichen oder tierischen Körpers vorgesehen ist, wobei die Stentstruktur einen expandierten Zustand, in dem sie an die Innenwandung des Blutgefäßes anliegt, und einen kontrahierten Zustand aufweist, in dem sie innerhalb eines Mikrokatheters durch das Blutgefäß bewegbar ist, wobei die Stentstruktur, vorzugsweise an ihrem proximalen Ende, mit einer Einführhilfe verbunden ist.

Gefäßendoprothesen, sog. Stents, werden häufig zur Behandlung von Gefäßverengungen eingesetzt und an der Stelle der Gefäßverengung dauerhaft implantiert, um das Gefäß offen zu halten. Typischerweise weisen Stents eine Röhrenstruktur auf und sind entweder lasergeschnitten, so dass sich eine Oberfläche aus Streben ergibt, zwischen denen Öffnungen vorliegen, oder bestehen aus einem Drahtgeflecht. Stents können durch einen Katheter an den Zielort gebracht und dort expandiert werden; im Falle selbstexpandierender Stents aus Formgedächtnismaterialien erfolgt diese Expansion und die Anlegung an die Gefäßinnenwand selbständig. Alternativ können Stents auch mit Hilfe von Ballons, auf die der Stent aufgecrimpt ist, oder anderen mechanischen Verfahren aufgeweitet werden. Nach endgültiger Platzierung verbleibt nur der Stent selbst am Zielort; Katheter, Führungsdrähte und andere Hilfsmittel werden aus dem Blutgefäßsystem entfernt.

Grundsätzlich ähnlich aufgebaute Implantate werden auch zum Verschließen von Aneurysmen eingesetzt, indem sie vor dem Hals eines Aneurysmas platziert werden. Derartige Flow Diverter haben allerdings in der Regel eine höhere Oberflächendichte als Stents zur Beseitigung von Stenosen. Ein Beispiel für einen Flow Diverter wird in der Anmeldung WO 2008/107172 A1 beschrieben. DE10301850 A1 zeigt einen medizinischen Stent zur Schienung und/oder Aufweitung röhrenförmiger Hohlorgane mit einem mit Durchbrechungen versehenen Grundkörper, welcher insbesondere bei der Behandlung von arteriovenösen Fehlbildungen zum Einsatz kommt.

Unter einem Vasospasmus versteht man eine krampfartige Verengung eines Blutgefäßes. Hiermit ist die Gefahr verbunden, dass nachfolgende Gefäße nicht mehr in ausreichendem Maße mit Blut versorgt werden (Ischämie), was zur Nekrose des durch die Gefäße mit Blut versorgten Gewebes führen kann. Gerade im zerebralen Bereich kann ein Vasospasmus einige Tage nach einer subarachnoidalen Blutung auftreten. Insofern ist ein Vasospasmus einer der Hauptgründe für nach einer Ruptur eines Aneurysmas und/oder Blutung aus demselben oder einer Operation in diesem Bereich auftretende Schlaganfälle bis hin zu Todesfällen.

Üblicherweise wird ein Vasospasmus medikamentös behandelt, wobei insbesondere Calciumkanalblocker oder Medikamente zum Einsatz kommen, die den NO-Level im Blut erhöhen. Ein Beispiel für einen Calciumkanalblocker ist Nimodipin, welches häufig nach subarachnoidalen Blutungen eingesetzt wird, um Vasospasmen vorzubeugen. Die medikamentöse Behandlung ist jedoch mit nicht unerheblichen Nebenwirkungen behaftet und darüber hinaus kosten- und zeitintensiv.

Es stellte sich somit die Aufgabe, Mittel zur Verfügung zu stellen, die die Behandlung eines Vasospasmus in anderer Art und Weise erlauben.

Diese Aufgabe wird erfindungsgemäß gelöst durch eine Vorrichtung mit einer Stentstruktur, die zur Einbringung in Blutgefäße des menschlichen oder tierischen Körpers vorgesehen ist, wobei die Stentstruktur einen expandierten Zustand, in dem sie an die Innenwandung des Blutgefäßes anliegt, und einen kontrahierten Zustand aufweist, in dem sie innerhalb eines Mikrokatheters durch das Blutgefäß bewegbar ist, wobei die Stentstruktur, vorzugsweise an ihrem proximalen Ende, mit einer Einführhilfe verbunden ist und sich die Stentstruktur aus einzelnen, miteinander verbundenen Streben oder aus einzelnen, eine Maschenstruktur ausbildenden Drähten zusammensetzt, wobei die Vorrichtung zur Behandlung eines Vasospasmus einsetzbar ist und die Stentstruktur einen proximalen, einen mittleren und einen distalen Abschnitt aufweist, wobei der proximale Abschnitt das proximale Ende umfasst, an dem die Stentstruktur mit der Einführhilfe verbunden ist, und wobei die expandierte Stentstruktur außerhalb des proximalen Endes über die Wirklänge im Wesentlichen eine konstante Radialkraft ausübt, wobei die Wirklänge den Teil der Stentstruktur definiert, der im expandierten Zustand an der Innenwandung des Blutgefäßes anliegt, wobei die Streben oder Drähte im proximalen und distalen Abschnitt einen größeren Querschnitt aufweisen als im mittleren Abschnitt und/oder die Dichte der Streben oder Drähte im proximalen und distalen Abschnitt höher ist als im mittleren Abschnitt.

Überraschend hat sich gezeigt, dass stentartige Strukturen in der Lage sind, einen Vasospasmus zu behandeln. Im Gegensatz zu den zuvor genannten Einsatzmöglichkeiten eines Stents zur Behandlung von Stenosen oder Aneurysmen verbleibt die Stentstruktur jedoch nicht dauerhaft im Blutgefäß, wird also nicht implantiert, sondern nur vorübergehend eingesetzt und nach wenigen Minuten wieder entfernt. Eine Ablösestelle zwischen Stentstruktur und Einführhilfe ist also nicht erforderlich.

Vorzugsweise handelt es sich um eine Stentstruktur, die selbstexpandierend ist und nach Freisetzung aus dem Mikrokatheter selbständig in den expandierten Zustand übergeht. Hierzu ist eine Stentstruktur aus einem Material mit Formgedächtniseigenschaften von Vorteil, insbesondere hat sich die Verwendung von Nickel-Titan-Legierungen bewährt, die unter dem Namen Nitinol bekannt sind. Denkbar sind jedoch auch Polymere mit Formgedächtniseigenschaften oder andere Legierungen.

Die erfindungsgemäße Vorrichtung kann insbesondere im neurovaskulären Bereich eingesetzt werden, möglich ist jedoch auch der Einsatz im kardiovaskulären oder peripheren Bereich.

Bei der Einführhilfe handelt es sich typischerweise um einen Einführdraht, auch Führungsdraht genannt. In ähnlicher Weise werden solche Einführdrähte auch für Implantate verwendet; allerdings ist bei Implantaten, die für den dauerhaften Verbleib im Gefäßsystem vorgesehen sind, der Einführdraht über eine Ablösestelle mit dem Implantat verbunden, wobei die Ablösestelle für eine mechanische, thermische oder elektrolytische Ablösung vorgesehen sein kann. Die Vorrichtung gemäß der Erfindung wird hingegen nur temporär an die Stelle des Vasospasmus gebracht, wo eine Expansion der Stentstruktur erfolgt. Die Einführhilfe ist bevorzugt aus Edelstahl, Nitinol oder einer Cobalt-Chrom-Legierung gefertigt.

Die Einführhilfe bzw. der Einführdraht ist bevorzugt am proximalen Ende der Stentstruktur radial außen angebracht. Mit anderen Worten befindet sich die Verbindung von Einführhilfe und Stentstruktur nicht im Zentrum der Stentstruktur, sondern exzentrisch an oder nahe der Gefäßinnenwandung. Auf diese Weise wird der Blutfluss möglichst wenig behindert. Die exzentrische Anordnung der Einführhilfe erleichtert darüber hinaus das Rückziehen der Vorrichtung in den Mikrokatheter.

Möglich ist auch, die Einführhilfe an mehreren Punkten mit der Stentstruktur, bevorzugt mit dem proximalen Ende der Stentstruktur, zu verbinden. Mehrere Verbindungspunkte mit der Stentstruktur bewirken auf der einen Seite eine geringfügig stärkere Behinderung des Blutflusses aufgrund der zusätzlichen, im Zentrum des Blutgefäßes verlaufenden Streben oder Drähte. Auf der anderen Seite kann das proximale Ende der Stentstruktur, die zur Einführhilfe hin ausläuft und daher nicht mehr vollständig an der Gefäßinnenwand anliegt und im Wesentlichen keine Radialkräfte auf diese ausüben kann, kürzer gehalten werden. Bei einer Einführhilfe, die an mehreren Punkten mit der Stentstruktur verbunden ist, verläuft die Einführhilfe in der Regel zentraler.

In der Regel erfolgt die Behandlung in der Weise, dass die erfindungsgemäße Vorrichtung innerhalb eines Mikrokatheters an den Zielort, d. h. den Ort des Vasospasmus vorgeschoben wird. Durch Zurückziehen des Mikrokatheters in Richtung proximal wird anschließend die Stentstruktur freigesetzt, die daraufhin expandiert und sich an die Innenwandung des Gefäßes anlegt und den Vasospasmus behandelt. An dieser Stelle wird die Stentstruktur für eine kurze Zeit belassen, typischerweise 1 bis 10 min. Anschließend wird der Mikrokatheter wieder in Richtung distal bewegt, um die Stentstruktur einzufalten, und der Mikrokatheter mitsamt der Vorrichtung zurückgezogen. Die Behandlung kann an mehreren aufeinanderfolgenden Tagen wiederholt werden.

Die Begriffe "proximal" und "distal" sind so zu verstehen, dass beim Einbringen der Vorrichtung zum behandelnden Arzt weisende Teile als proximal, vom behandelnden Arzt weg weisende Teile als distal bezeichnet werden. Die Vorrichtung wird somit typischerweise durch einen Mikrokatheter in distaler Richtung vorgeschoben. Der Begriff "axial" bezieht sich auf die von proximal nach distal verlaufende Längsachse der Vorrichtung, der Begriff "radial" auf hierzu senkrechte Ebenen.

Parallel zu dieser Behandlung mit Hilfe der erfindungsgemäßen Vorrichtung kann auch eine medikamentöse Behandlung erfolgen, beispielsweise mit Nimodipin. Dieses kann insbesondere intra-arteriell an der Stelle des Vasospasmus appliziert werden.

Grundsätzlich kann sich die Stentstruktur aus einzelnen, miteinander verbundenen Stegen bzw. Streben zusammensetzen. Eine solche Stentstruktur kann durch Laserschneiden in grundsätzlich bekannter Weise hergestellt werden. Zusätzlich ist es sinnvoll, die Stentstruktur einer Elektropolitur zu unterziehen, um diese glatter und abgerundeter und damit weniger traumatisch werden zu lassen. Darüber hinaus sinkt die Gefahr der Anhaftung von Keimen oder sonstiger Verunreinigungen.

Alternativ kann die Stentstruktur auch eine Maschenstruktur aus einzelnen Drähten sein, die ein Geflecht ausbilden. Die Drähte verlaufen hierbei typischerweise helixförmig entlang der Längsachse, wobei gegenläufig verlaufende Drähte an den Kreuzungspunkten über- und untereinander her verlaufen, sodass sich wabenförmige Öffnungen zwischen den Drähten ausbilden. Die Gesamtzahl der Drähte beträgt bevorzugt 8 bis 64. Bei den Drähten, die die Maschenstruktur ausbilden, kann es sich um einzelne Drähte aus Metall handeln, möglich ist aber auch das Vorsehen von Litzen, d. h. mehreren Drähten geringen Durchmessers, die zusammen ein Filament bilden und vorzugsweise miteinander verdrillt sind.

Ein Vorteil einer Stentstruktur aus miteinander verbundenen Stegen oder Streben, die insbesondere durch Laserschneiden erzeugt wird, gegenüber einer Maschenstruktur aus Drähten ist darin zu sehen, dass eine Stentstruktur aus Streben bei Expansion weniger zur Längenkontraktion neigt als eine Maschenstruktur. Die Längenkontraktion sollte möglichst klein gehalten werden, da die Stentstruktur während einer Längenkontraktion die umgebende Gefäßwand zusätzlichem Stress aussetzt. Da ein Vasospasmus letztlich gerade auf Reize, die auf das Gefäß ausgeübt werden, zurückzuführen ist, ist eine zusätzliche Belastung bei der Vasospasmusbehandlung zu vermeiden.

Eine Stentstruktur aus miteinander verbundenen Streben ist auch insofern vorteilhaft, weil die von einer solchen Stentstruktur bei im Übrigen vergleichbarem Aufbau, Streben-/Drahtdichte und Streben-/Drahtstärke die von der Stentstruktur ausgeübte Radialkraft höher ist als bei einer aus Drähten aufgebauten Maschenstruktur. Der Grund liegt darin, dass die Streben an den Schnittpunkten eine feste Verbindung aufweisen, während die Drähte einer Maschenstruktur in der Regel nur über- und untereinander her verlaufen.

Die Streben oder Drähte können einen runden, ovalen, quadratischen oder rechteckigen Querschnitt aufweisen, wobei im Falle eines quadratischen oder rechteckigen Querschnitts eine Abrundung der Kanten von Vorteil ist.

Die Öffnungen, die sich in der Stentstruktur zwischen den einzelnen Streben oder Drähten ausbilden, sollten einen einbeschriebenen Durchmesser von 0,1 bis 6 mm aufweisen, wobei unter dem einbeschriebenen Durchmesser der Durchmesser des größtmöglichen Kreises verstanden wird, der in der Öffnung platziert werden kann. Die Angaben beziehen sich auf die Stentstruktur im expandierten Zustand, d. h. den Zustand, den die Stentstruktur annimmt, wenn sie keinen äußeren Zwängen ausgesetzt ist.

Bevorzugt sind Öffnungen mit einem einbeschriebenen Durchmesser von ≥ 1 mm, d. h. eine relativ grobmaschige Stentstruktur, da diese eine Radialkraft in geeigneter Größenordnung zur Behandlung eines Vasospasmus ausüben kann. Beispielsweise kann eine Stentstruktur mit einem Durchmesser im expandierten Zustand von 3 bis 5 mm einen einbeschriebenen Durchmesser der Öffnungen von 2 bis 4,5 mm aufweisen.

Die sich in der Stentstruktur ergebenden Öffnungen sollten rundum geschlossen, d. h. ohne Unterbrechungen von Streben bzw. Drähten umgeben sein (sog. "Closed-Cell-Design"), da dies das Befördern der Stentstruktur nach der Behandlung in den Mikrokatheter durch Aufschub desselben erleichtert.

Sinnvoll ist darüber hinaus zur Erzeugung einer Radialkraft in der richtigen Größenordnung die Verwendung von Streben oder Drähten mit einem verhältnismäßig großen Querschnitt bzw. Durchmesser, d. h. die Verwendung von relativ massiven Streben/Drähten. So haben sich bei Verwendung von Streben oder Drähten mit einem im Wesentlichen rechteckigen Querschnitt eine Höhe und eine Breite der Streben/Drähte von 30 bis 300 μm als vorteilhaft herausgestellt, wobei auch ein rechteckiger Querschnitt mit abgerundeten Kanten als im Wesentlichen rechteckig aufgefasst wird. Im Falle eines runden Querschnitts sollte der Durchmesser zwischen 30 und 300 μm liegen.

In der Regel ist die Stentstruktur am proximalen Ende offen ausgebildet. Am distalen Ende kann die Stentstruktur ebenfalls offen, aber auch geschlossen sein. Eine beidseitig offene Stentstruktur hat den Vorteil, dass der Blutfluss möglichst wenig gestört und einer Unterversorgung nachfolgender Blutgefäße und des durch diese versorgten Gewebes vorgebeugt wird. Auf der anderen Seite ist eine geschlossene Struktur am distalen Ende atraumatischer. Unter offen wird dabei verstanden, dass am jeweiligen Ende der Stentstruktur keine Streben oder Drähte vorhanden sind und sich Streben/Drähte auf den äußeren Umfang der Stentstruktur beschränken. Bei einem geschlossenen Ende hingegen liegen auch im Zentrum der Stentstruktur Streben oder Drähte vor. Da zwischen den Streben oder Drähten Öffnungen vorhanden sind, ist auch bei einem geschlossenen distalen Ende dieses Ende nicht vollständig dicht; der Blutfluss kann weiterhin durch die Öffnungen hindurch erfolgen.

Sinnvoll ist eine antithrombogene Beschichtung auf der Innenseite der Stentstruktur, weil diese über eine gewisse Zeit im Blutgefäß verbleibt und währenddessen die Bildung eines Thrombus im ohnehin durch das Auftreten des Vasospasmus verengten Blutgefäß vermieden werden muss. Auf der Außenseite der Stentstruktur ist eine gefäßrelaxierende Beschichtung von Vorteil, beispielsweise mit einem Calciumkanalblocker wie Nimodipin.

Auch wenn eine antithrombogene Beschichtung insbesondere auf der Innenseite der Stentstruktur und eine gefäßrelaxierende Beschichtung insbesondere auf der Außenseite der Stentstruktur sinnvoll sind, können die antithrombogene oder die gefäßrelaxierende Beschichtung auch insgesamt auf der Stentstruktur aufgebracht sein bzw. die Streben/Drähte können auf allen Seiten eine entsprechende Beschichtung aufweisen. In diesem Fall ist die Beschichtung nicht auf die Innen- oder Außenseite der Stentstruktur beschränkt.

Allgemein ist zu sämtlichen Beschichtungen zu sagen, dass diese auch jeweils nur einen Teil bzw. eine Teillänge der Stentstruktur betreffen können. Von Bedeutung ist eine Beschichtung insbesondere in den Bereichen der Stentstruktur, die die Gefäßinnenwandung berühren, d. h. im Wesentlichen der zylindrische Teil der Stentstruktur.

Die durch die expandierte Stentstruktur nach radial außen auf die Gefäßinnenwand einwirkende Kraft sollte zwischen 2 und 16 N/m, bevorzugt zwischen 5 und 8 N/m liegen. Die Angabe der Radialkraft bezieht sich auf die pro Längeneinheit radial ausgeübte Kraft, d. h. es handelt sich um die relative Radialkraft. Dabei wird nur der Teil der Stentstruktur berücksichtigt, der an der Gefäßinnenwand anliegt und daher in der Lage ist, Kräfte auf diese auszuüben (Wirklänge). Entlang der Wirklänge muss die Stentstruktur mindestens 50 % einer um die Stentstruktur gezogenen Hülle bedecken. Im Gegensatz dazu bezeichnet die absolute Radialkraft den Gesamtwert der Stentstruktur.

Die Bestimmung der von der Stentstruktur ausgeübten Radialkraft (Chronic Outward Force, COF) erfolgt in folgender Weise mittels V-Block-Test:

Der Testaufbau des V-Block-Tests besteht aus zwei Polymethylmethacrylat (PMMA)-Blöcken, welche jeweils mit einer eingefrästen und glatt polierten 90° V-Nut versehen sind. Diese V-Blöcke werden so übereinander platziert, dass bei Kontakt der Blöcke ein Hohlraum mit quadratischem Querschnitt entsteht. Während einer der V-Blöcke fixiert ist, ist der andere mit einem Kraftsensor versehen.

Die COF beschreibt die Kraft, die die Stentstruktur bei seiner Selbstexpansion auf das Blutgefäß bzw. im Test die V-Blöcke ausübt. Zur Ermittlung der Radialkraft wird die Stentstruktur innerhalb eines Transportschlauchs oder eines Mikrokatheters mittig zwischen die V-Blöcke positioniert. Anschließend wird der Transportschlauch/Mikrokatheter zurückgezogen und die Stentstruktur freigesetzt. Wegen ihrer selbstexpandierenden Eigenschaften weitet sie sich aus und die von der Stentstruktur ausgehende Radialkraft kann über den mit einem der V-Blöcke verbundenen Kraftsensor gemessen und ausgewertet werden. Um Stentstrukturen unterschiedlicher Länge vergleichen zu können, wird die relative Radialkraft errechnet:

$$COFrel. = \frac{COFabs.}{Wirklänge\ der\ Stentstruktur}$$

Die von der Stentstruktur im expandierten Zustand über die Länge ausgeübte Radialkraft ist im Wesentlichen konstant, d. h. im proximalen Abschnitt und im distalen Abschnitt entspricht die Radialkraft der des mittleren Abschnitts. Bei herkömmlichen, gleichmäßig aufgebauten Stents ist hingegen die tatsächlich wirkende Radialkraft im proximalen und distalen Abschnitt zumeist schwächer als im mittleren Abschnitt. Die Radialkraft wird daher im proximalen und distalen Abschnitt gezielt erhöht, um eine Stentstruktur zu erzeugen, bei der die Radialkraft im expandierten Zustand über die Wirklänge im Wesentlichen konstant ist, wobei das proximale Ende der Stentstruktur, an dem die Streben oder Drähte typischerweise nicht mehr vollständig an der Gefäßinnenwand anliegen, für die Radialkraft unberücksichtigt bleibt. Das proximale Ende bezeichnet somit den am weitesten proximal liegenden Teil der Stentstruktur, der nicht mehr zur Wirklänge gehört und in dem die Streben/Drähte auf die Einführhilfe hin zulaufen. Eine typische Länge dieses proximalen Endes beträgt 8 bis 10 mm, d. h. die Gesamtlänge der Stentstruktur ist ungefähr um diesen Betrag länger als die Wirklänge der Stentstruktur.

Um die verstärkte Radialkraft im proximalen und distalen Abschnitt herbeizuführen, können die Streben oder Drähte hier einen größeren Querschnitt aufweisen als im mittleren Abschnitt. Die Streben/Drähte sind somit massiver ausgebildet, wodurch die grundsätzliche Tendenz einer Stentstruktur, im mittleren Abschnitt höhere Radialkräfte auszuüben, ganz oder teilweise kompensiert wird.

Alternativ oder zusätzlich kann die Dichte der Streben oder Drähte im proximalen und distalen Abschnitt höher sein als im mittleren Abschnitt. Auch durch diese Maßnahme wird der bei herkömmlichen Stents zu beobachtende Abfall der Radialkraft nach proximal bzw. distal ganz oder teilweise kompensiert.

Möglich ist es auch, die Stentstruktur mit einem Schlitz zu versehen, der sich wendelförmig über die Mantelfläche der Stentstruktur oder in Längsrichtung entlang der Mantelfläche der Stentstruktur erstreckt. Dabei können einzelne Streben oder Drähte den Schlitz überspannen, um so den Radialkraftverlauf zu beeinflussen.

Der Durchmesser der Stentstruktur im expandierten Zustand liegt typischerweise im Bereich von 2 bis 8 mm, vorzugsweise im Bereich von 4 bis 6 mm. Die Gesamtlänge der Stentstruktur im expandierten Zustand beträgt in der Regel 5 bis 50 mm, bevorzugt 10 bis 45 mm, weiter bevorzugt 20 bis 40 mm. Die Wirklänge, d. h. die Länge der Stentstruktur im expandierten Zustand, die tatsächlich radiale Kräfte auf die Gefäßinnenwand ausübt, ist zumeist ca. 8 bis 10 mm kürzer.

Sinnvollerweise verfügt die Vorrichtung über ein oder mehrere röntgendichte Markierungen, um dem behandelnden Arzt eine Visualisierung zu ermöglichen. Die röntgendichten Markierungen können z. B. aus Platin, Palladium, Platin-Iridium, Tantal, Gold, Wolfram oder anderen röntgendichten Metallen sein. Beispielsweise können röntgendichte Wendeln an verschiedenen Punkten der Vorrichtung angebracht sein. Möglich ist auch, die Stentstruktur, insbesondere die Streben oder Drähte der Stentstruktur mit einer Beschichtung aus einem röntgendichten Material zu versehen, beispielsweise mit einer Goldbeschichtung. Diese kann z. B. eine Stärke von 1 bis 6 $\mu$m aufweisen. Die Beschichtung mit einen röntgendichten Material muss nicht die gesamte Stentstruktur umfassen; von Bedeutung ist sie insbesondere in den Bereichen der Stentstruktur, die die Gefäßinnenwandung berühren, d. h. im Wesentlicher im zylindrischen Teil der Stentstruktur. Auch beim Vorsehen einer röntgendichten Beschichtung kann es allerdings sinnvoll sein, zusätzlich einen oder mehrere röntgendichte Markierungen an der Vorrichtung anzubringen, insbesondere am distalen Ende der Stentstruktur.

Bei dem Verfahren zur Vasospasmusbehandlung wird die Stentstruktur der Vorrichtung mittels der Einführhilfe an die Position des Vasospasmus gebracht und dort expandiert, was in der Regel durch Zurückziehen des Mikrokatheters, in dem die Vorrichtung untergebracht ist, in Richtung proximal erfolgt. An der Position des Vasospasmus wird die Stentstruktur für einige Minuten, bevorzugt 1 bis 10 min belassen. Anschließend wird die Stentstruktur aus dem Blutgefäß entfernt. Hierzu kann der Mikrokatheter in Richtung distal vorgeschoben werden, um die Stentstruktur erneut einzufalten und im Mikrokatheter aufzunehmen. Mikrokatheter und Vorrichtung können anschließend zurückgezogen und aus dem Blutgefäßsystem entfernt werden. Sinnvollerweise wird die geschilderte Behandlung an mehreren aufeinanderfolgenden Tagen wiederholt, um die Vasospasmusbehandlung fortzusetzen.

Sämtliche Ausführungen, die bezüglich der Vorrichtung gemacht wurden, gelten in gleicher Weise auch für das Verfahren und umgekehrt.

Die Erfindung wird durch die beiliegenden Abbildungen beispielhaft näher erläutert. Es zeigen:

Figur 1a      Eine erfindungsgemäße Vorrichtung in der Seitenansicht;

Figur 1b      die erfindungsgemäße Vorrichtung aus Figur 1a in einer abgewickelten Darstellung;

Figur 2      eine Darstellung des Radialkraftniveaus entlang der Stentstruktur;

Figur 3      eine Messapparatur zur Ermittlung der von der Stentstruktur ausgeübten Radialkraft;

Figur 4      die Ermittlung der Wirklänge der Stentstruktur und

Figur 5      eine bevorzugte Ausführungsform der Stentstruktur in der Seitenansicht.

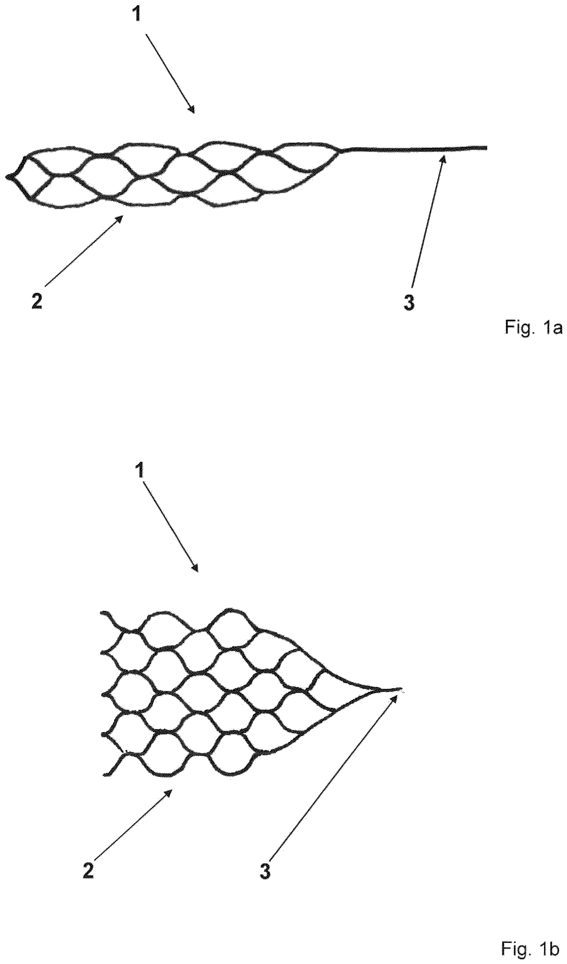

In Figur 1a ist die erfindungsgemäße Vorrichtung 1 in der Seitenansicht dargestellt. Die Vorrichtung weist eine Stentstruktur 2 und eine Einführhilfe 3 in Form eines Einführdrahtes auf. Die Stentstruktur 2 ist in diesem Beispiel lasergeschnitten und setzt sich aus Streben zusammen, die insgesamt eine kontinuierliche Wabenstruktur ergeben. Die Einführhilfe 3 ist exzentrisch, d. h. im Randbereich mit der Stentstruktur 2 an deren proximalem Ende verbunden.

In Figur 1b ist die Vorrichtung 1 aus Figur 1a abgewickelt dargestellt, d. h. die hypothetische Situation, die sich ergeben würde, wenn die im Wesentlichen zylindrische Stentstruktur 2 aus Figur 1a entlang der Längsachse aufgeschnitten und flach ausgebreitet würde. Die Einführhilfe 3 ist hier nur verkürzt abgebildet.

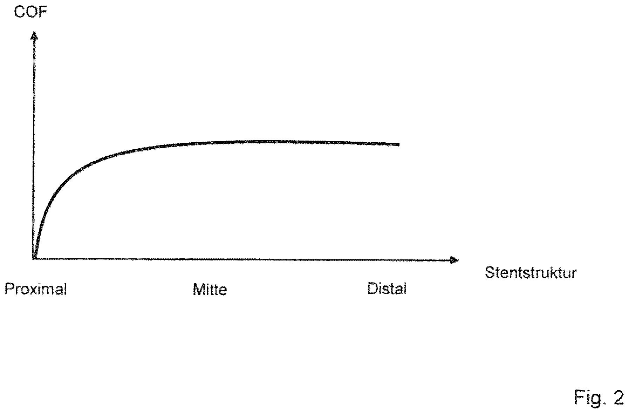

In Figur 2 ist der Verlauf der Radialkraft (COF) entlang der Stentstruktur 2 dargestellt. Lediglich am proximalen Ende wirkt eine geringere Radialkraft auf die umliegende Gefäßinnenwand, im Übrigen ist die Radialkraft über die gesamte Stentstruktur 2 konstant. Das Absinken der Radialkraft am proximalen Ende hängt damit zusammen, dass hier die Stentstruktur 2 zur Einführhilfe 3 in einem Punkt zusammenläuft und die Stentstruktur 2 daher nicht den gesamten Umfang zur Gefäßinnenwand bedeckt.

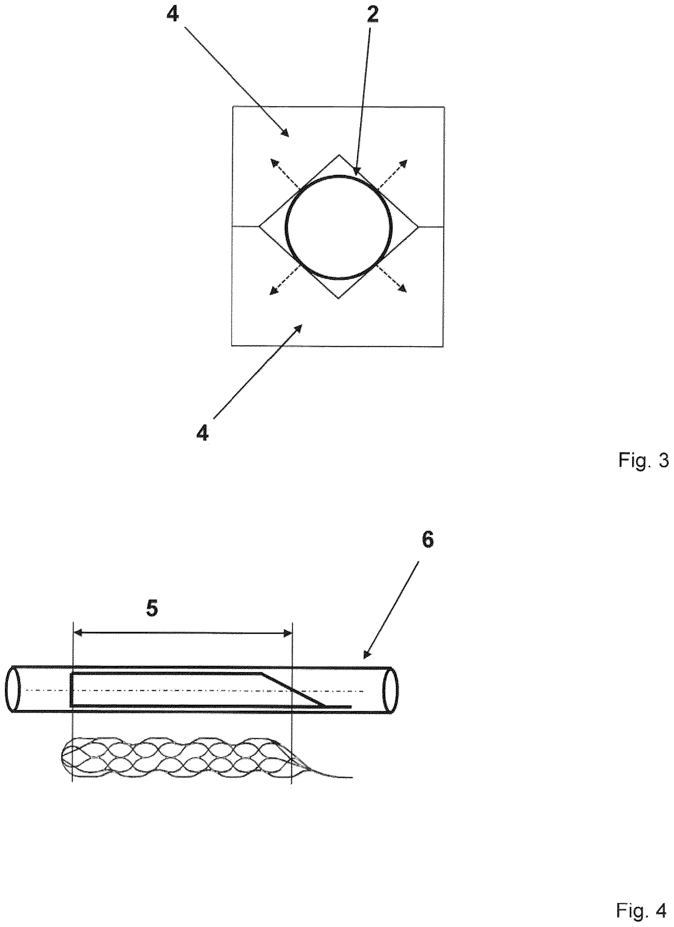

In Figur 3 wird eine Vorrichtung zur Ermittlung der Radialkraft mittels V-Block-Test für die Stentstruktur 2 dargestellt. Die Vorrichtung zur Ermittlung der Radialkraft weist 2 V-Blöcke 4 auf, die jeweils mit einer 90° V-Nut versehen sind. Bei Aufeinandersetzen der V-Blöcke 4 entsteht somit ein Hohlraum mit quadratischem Querschnitt. Während der untere V-Block 4 fixiert ist, weist der obere V-Block 4 einen Kraftsensor auf, um die Radialkraft zu ermitteln.

Die zu testende Stentstruktur 2 wird in den quadratischen Hohlraum zwischen den V-Blöcken 4 zunächst innerhalb eines Transportschlauchs oder Mikrokatheters eingelegt. Anschließend wird der Schlauch/Mikrokatheter zurückgezogen, sodass die selbstexpandierende Stentstruktur 2 zu den Flächen der V-Blöcke 4 expandieren kann. Die von der Stentstruktur 2 ausgeübten Kräfte sind durch die gestrichelten Pfeile angedeutet und werden über die V-Blöcke 4 zum Kraftsensor übertragen und ausgewertet.

Wie bereits erwähnt, wird, um Stentstrukturen 2 unterschiedlicher Länge miteinander vergleichen zu können, die relative Radialkraft verwendet, d. h. die absolute Radialkraft bezogen auf die Wirklänge 5 der Stentstruktur 2. Zur Ermittlung dieser Wirklänge 5 wird, wie in Figur 4 dargestellt, die Stentstruktur in ein transparentes Rohr 6 mit rundem Querschnitt gezogen. Die Wirklänge 5 ist die Länge, in der die Stentstruktur 2 mindestens 50 % des Umfangs bedeckt. Insbesondere das proximale Ende der Stentstruktur 2, an dem diese zur Einführhilfe hin ausläuft, gehört daher nicht zur Wirklänge 5.

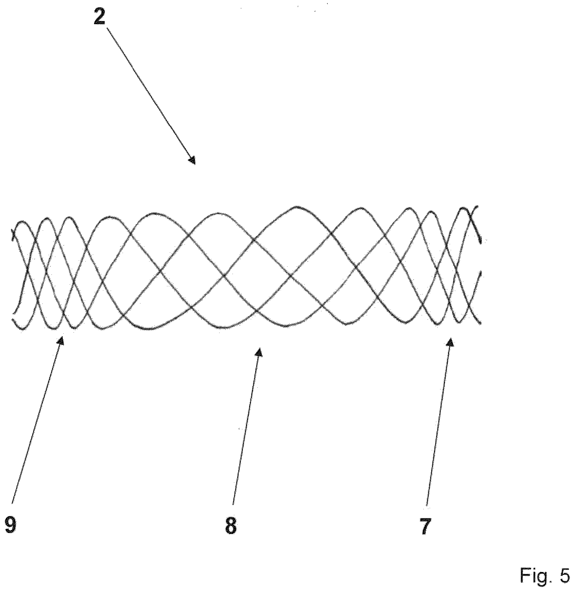

In Figur 5 schließlich wird eine bevorzugte Ausführungsform einer erfindungsgemäßen Stentstruktur 2 in der Seitenansicht dargestellt, wobei sich die Stentstruktur 2 in einen proximalen Abschnitt 7, einen mittleren Abschnitt 8 und einen distalen Abschnitt 9 gliedert. Die Darstellung beschränkt sich auf die Wirklänge 5, d. h. weder das proximale Ende noch die Einführhilfe sind abgebildet. Im Gegensatz zu Figur 1 handelt es sich hier um eine Stentstruktur 2 aus einzelnen, eine Maschenstruktur ausbildenden Drähten. Um, wie in Figur 2 dargestellt, einen gleichmäßigen Radialkraftverlauf über die gesamte Wirklänge 5 zu erreichen, wird die Dichte der Maschenstruktur sowohl im proximalen Abschnitt 7 als auch im distalen Abschnitt 9 gegenüber dem mittleren Abschnitt 8 erhöht. Der bei gleichmäßig aufgebauten Stentstrukturen 2 regelmäßig zu beobachtende Abfall der Radialkraft nach proximal und distal wird somit durch die höhere Dichte an Drähten kompensiert.

**Patentansprüche**

1. Vorrichtung mit einer Stentstruktur (2), die zur Einbringung in Blutgefäße des menschlichen oder tierischen Körpers vorgesehen ist, wobei die Stentstruktur (2) einen expandierten Zustand, in dem sie an die Innenwandung des Blutgefäßes anliegt, und einen kontrahierten Zustand aufweist, in dem sie innerhalb eines Mikrokatheters durch das Blutgefäß bewegbar ist, wobei die Stentstruktur (2), vorzugsweise an ihrem proximalen Ende, mit einer Einführhilfe (3) verbunden ist und sich die Stentstruktur (2) aus einzelnen, miteinander verbundenen Streben oder aus einzelnen, eine Maschenstruktur ausbildenden Drähten zusammensetzt, wobei die Vorrichtung (1) zur Behandlung eines Vasospasmus einsetzbar ist und die Stentstruktur (2) einen proximalen, einen mittleren und einen distalen Abschnitt (7, 8, 9) aufweist, wobei der proximale Abschnitt (7) das proximale Ende umfasst, an dem die Stentstruktur (2) mit der Einführhilfe (3) verbunden ist, und wobei die expandierte Stentstruktur (2) außerhalb des proximalen Endes über die Wirklänge (5) im Wesentlichen eine konstante Radialkraft ausübt, wobei die Wirklänge (5) den Teil der Stentstruktur (2) definiert, der im expandierten Zustand an der Innenwandung des Blutgefäßes anliegt, **dadurch gekennzeichnet, dass** die Streben oder Drähte im proximalen und distalen Abschnitt (7, 9) einen größeren Querschnitt aufweisen als im mittleren Abschnitt (8) und/oder die Dichte der Streben oder Drähte im proximalen und distalen Abschnitt (7, 9) höher ist als im mittleren Abschnitt (8).

2. Vorrichtung nach Anspruch 1, **dadurch gekennzeichnet, dass** die Stentstruktur (2) selbstexpandierend ist und nach Freisetzung aus dem Mikrokatheter selbständig in den expandierten Zustand übergeht.

3. Vorrichtung nach Anspruch 1 oder 2, **dadurch gekennzeichnet, dass** die Stentstruktur (2) zwischen den einzelnen Streben oder Drähten Öffnungen aufweist, die im expandierten Zustand einen einbeschriebenen Durchmesser von 0,1 bis 6 mm aufweisen.

4. Vorrichtung nach einem der Ansprüche 1 bis 3, **dadurch gekennzeichnet, dass** die Streben oder Drähte im Falle eines im Wesentlichen rechteckigen Querschnitts eine Höhe und Breite von 30 bis 300 $\mu$m und im Falle eines runden Querschnitts einen Durchmesser von 30 bis 300 $\mu$m aufweisen.

5. Vorrichtung nach einem der Ansprüche 1 bis 4, **dadurch gekennzeichnet, dass** die Stentstruktur (2) am proximalen und/oder am distalen Ende im Zentrum keine Streben oder Drähte aufweist.

6. Vorrichtung nach einem der Ansprüche 1 bis 5, **dadurch gekennzeichnet, dass** die Stentstruktur (2) am distalen Ende im Zentrum Streben oder Drähte aufweist.

7. Vorrichtung nach einem der Ansprüche 1 bis 6, **dadurch gekennzeichnet, dass** die Stentstruktur (2) auf der Innenseite eine antithrombogene Beschichtung aufweist.

8. Vorrichtung nach einem der Ansprüche 1 bis 7, **dadurch gekennzeichnet, dass** die Stentstruktur (2) auf der Außenseite eine gefäßrelaxierende Beschichtung aufweist.

9. Vorrichtung nach einem der Ansprüche 1 bis 8, **dadurch gekennzeichnet, dass** die Stentstruktur (2) ganz oder teilweise über eine röntgendichte Beschichtung, insbesondere eine Goldbeschichtung verfügt.

10. Vorrichtung nach einem der Ansprüche 1 bis 9, **dadurch gekennzeichnet, dass** die von der expandierten Stentstruktur (2) nach radial außen wirkende Kraft 2 bis 16 N/m, bevorzugt 5 bis 8 N/m beträgt.


**Claims**

1. Device with a stent structure (2) which is intended for insertion into blood vessels of the human or animal body, wherein the stent structure (2) has an expanded state, in which it lies against the inner wall of the blood vessel, and a contracted state, in which it can be moved through the blood vessel within a microcatheter, wherein the stent structure (2) is connected, preferably at its proximal end, to an insertion aid (3) and the stent structure (2) is composed of individual struts connected to one another or of individual wires forming a mesh structure, wherein the device (1) can be used to treat a vasospasm and the stent structure (2) has a proximal, a middle and a distal section (7, 8, 9), wherein the proximal section (7) comprises the proximal end at which the stent structure (2) is connected to the insertion aid (3), and wherein the expanded stent structure (2) outside the proximal end exerts a substantially constant radial force over the effective length (5), wherein the effective length defines that part of the stent structure (2) which in the expanded state abuts against the inner wall of the blood vessel,
**characterized in that**
the struts or wires in the proximal and distal sections (7, 9) have a larger cross-section than in the middle section (8) and/or the density of the struts or wires in the proximal and distal section (7, 9) is higher than in the middle section (8).

2. Device according to claim 1, **characterized in that** the stent structure (2) is self-expanding and changes automatically to the expanded state after release from the microcatheter.

3. Device according to claim 1 or 2, **characterized in that** the stent structure (2) has openings between the individual struts or wires which have an inscribed diameter of 0.1 to 6 mm in the expanded state.

4. Device according to any one of claims 1 to 3, **characterized in that** the struts or wires have a height and width of 30 to 300 μm in the case of an essentially rectangular cross-section and a diameter of 30 to 300 μm in the case of a round cross-section.

5. Device according to any one of claims 1 to 4, **characterized in that** the stent structure (2) has no struts or wires at the proximal and/or distal end in the center.

6. Device according to any one of claims 1 to 5, **characterized in that** the stent structure (2) has struts or wires at the distal end in the center.

7. Device according to any one of claims 1 to 6, **characterized in that** the stent structure (2) has an antithrombogenic coating on the inner side.

8. Device according to any one of claims 1 to 7, **characterized in that** the stent structure (2) has a vascular-relaxing coating on the outside.

9. Device according to any one of claims 1 to 8, **characterized in that** the stent structure (2) has a radiopaque coating, in particular a gold coating, in whole or in part.

10. Device according to any one of claims 1 to 9, **characterized in that** the force acting radially outwards from the expanded stent structure (2) is 2 to 16 N/m, preferably 5 to.8 N/m.

**Revendications**

1. Dispositif avec une structure de stent (2), qui est prévue pour l'introduction dans des vaisseaux sanguins du corps humain ou animal, la structure de stent (2) présentant un état dilaté, dans lequel elle s'applique à la paroi intérieure du vaisseau sanguin, et un état contracté, dans lequel elle est mobile à l'intérieur d'un microcathéter à travers le vaisseau sanguin, la structure de stent (2), de préférence à son extrémité proximale, est reliée à une aide à l'introduction (3) et la structure de stent (2) se compose d'entretoises individuelles reliées entre elles ou de fils individuels formant une structure maillée, le dispositif (1) pouvant être utilisé pour traiter un vasospasme et la structure de stent (2) présentant une section proximale, une section médiane et une section distale (7, 8, 9), dans lequel la section proximale (7) comprend l'extrémité proximale à laquelle la structure de stent (2) est reliée à l'auxiliaire d'introduction (3), et dans lequel la structure de stent (2) expansée exerce une force radiale sensiblement constante sur la longueur active (5) en dehors de l'extrémité proximale, la longueur active (5) définissant la partie de la structure de stent (2) qui, à l'état expansé, est en contact avec la paroi interne du vaisseau sanguin, **caractérisé en ce que** les entretoises ou les fils présentent une section transversale plus grande dans les sections proximale et distale (7, 9) que dans la section centrale (8) et/ou la densité des entretoises ou des fils est plus élevée dans les sections proximale et distale (7, 9) que dans la section centrale (8).

2. Dispositif selon la revendication 1, **caractérisé en ce que** la structure de stent (2) est auto-expansible et passe automatiquement à l'état expansé après avoir été libérée du microcathéter.

3. Dispositif selon la revendication 1 ou 2, **caractérisé en ce que** la structure de stent (2) présente, entre les différentes entretoises ou fils, des ouvertures qui, à l'état expansé, présentent un diamètre inscrit de 0,1 à 6 mm.

4. Dispositif selon l'une des revendications 1 à 3, **caractérisé en ce que** les entretoises ou les fils ont une hauteur et une largeur de 30 à 300 μm dans le cas d'une section transversale sensiblement rectangulaire et un diamètre de 30 à 300 μm dans le cas d'une section transversale circulaire.

5. Dispositif selon l'une quelconque des revendications 1 à 4, **caractérisé en ce que** la structure de stent (2) ne comporte pas d'entretoises ou de fils au centre de l'extrémité proximale et/ou de l'extrémité distale.

6. Dispositif selon l'une quelconque des revendications 1 à 5, **caractérisé en ce que** la structure de stent (2) comporte des entretoises ou des fils à l'extrémité distale au centre.

7. Dispositif selon l'une des revendications 1 à 6, **caractérisé en ce que** la structure de stent (2) présente un revêtement antithrombogène sur la face interne.

8. Dispositif selon l'une des revendications 1 à 7, **caractérisé en ce que** la structure de stent (2) présente un revêtement vasorelaxant sur le côté extérieur.

9. Dispositif selon l'une des revendications 1 à 8, **caractérisé en ce que** la structure de stent (2) dispose entièrement ou partiellement d'un revêtement radio-opaque, en particulier d'un revêtement en or.

10. Dispositif selon l'une des revendications 1 à 9, **caractérisé en ce que** la force agissant radialement vers l'extérieur par la structure de stent expansée (2) est de 2 à 16 N/m, de préférence de 5 à 8 N/m.

Fig. 1a

Fig. 1b

Fig. 2

EP 3 463 206 B1

Fig. 3

Fig. 4

12

Fig. 5

**EP 3 463 206 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2008107172 A1

- DE 10301850 A1